# EUROPEAN PATENT APPLICATION

(11) **EP 4 789 778 A1**
(43) Date of publication of application: **12.08.2026**
(21) Application number: 25770411.4
(22) Date of filing: 13.08.2025
(51) Int. Cl.: B06B 1/04, A61H 23/02, A61N 2/00

(54) **MATTRESS FOR IMPROVING BLOOD CIRCULATION AND AIDING SOUND SLEEP BY GENERATING MAGNETIC FIELD AND WAVELENGTHS**

(30) Priority: 16.08.2024 KR 20240109538
(71) Applicant: Shoealls Co, Ltd., Cheonan-si, Chungcheongnam-do 31470 (KR)
(72) Inventor: LEE, Cheonggn, Cheonan-si, Chungcheongnam-do 31066 (KR)
(74) Representative: Valet Patent Services Limited
(86) International application number: PCT/KR2025/012286
(87) International publication number: WO 2026/038881

(57) **Abstract**

The present invention exhibits the effect of improving the health of a user by using a vibration device arranged in multiple rows built into the mattress of a bed.

## Description

### TECHNICAL FIELD

The present invention relates to a mattress or bed mounted with a vibration device.

### BACKGROUND

Beds or mattresses that promotes deep sleep and fatigue recovery include, for example, germanium beds, stone beds, jade beds, illite beds, and loess beds. These products add minerals or elements such as germanium to the mattress or mattress interior material, or add loess. However, these products have limitations in terms of improving health in that they penetrate various materials and indirectly affect the human body.

Meanwhile, a technology is known in which a vibration device is built into the sole of a shoe, and if a wearer walks while wearing the shoe, the vibration elements within the vibration device vibrates due to the impact, and this vibration is transmitted to the sole of the wearer's foot. Since most of the human nerves and blood vessels pass through the sole of the foot, stimulating, vibrating, or applying acupressure to the soles of the foot can improve health.

In regard to vibration devices for shoes, the applicant has filed numerous patent applications, including Korean Patent Registration No. 10-1978880, Korean Patent Registration No. 10-2037241, and Korean Patent Registration No. 10-2494468. In Korean Patent Registration No. 10-2557301, the applicant disclosed a vibration unit mounted in a recess of a shoe middle sole. The vibration unit comprises a casing, a vibration frame, a first vibration plate, a second vibration plate and a vibration means. The first vibration plate has a structure in which the other end thereof is connected to the vibration frame and one end thereof is disposed in the receiving portion, and the second vibration plate has a structure in which the other end thereof is connected to the vibration frame and one end thereof is disposed in the receiving portion. Each of the first and second vibration plates comprises: a support connected to the vibration frame, a pair of branches branching into two from an end of the support; and a pair of discs formed at ends of the branch.

A U-shaped recess recessed inward is formed at a connection portion between each branch and each disc, and this is to facilitate a vertical amplitude motion of the disc. A pair of U-shaped neck grooves are formed at the connection portion between the vibration frame and each of the supports of the first and second vibration plates, and this is to facilitate a vertical amplitude motion of each of the vibration plates.

A movable magnet is mounted on the disc, an upper magnet is placed in the upper casing opposite to the movable magnet, and a lower magnet is placed in the lower casing. Therefore, the impact of external force from the shoe pushes the movable magnet with the repulsive force of the upper magnet, causing the vibration frame to move downward. This up-and-down movement of the vibration frame is repeated in minute cycles, transmitting periodic and uniform vibrations through the casing to the soles of the wearer's feet.

The above patents have been realized as actual products, demonstrating that the vibration effects are excellent.

However, the technology that applies vibration devices to mattresses or beds is not yet commercially available. Springs are mounted in the bed mattress to support the body and absorb vibrations, but they do not provide active vibrations. The elastic effect of the springs can only be felt when a significant amount of the person's body weight is applied, and its effect on improving health is minimal. Structures that attach magnets to beds, futons, or linoleum have been proposed to improve health, but these also only exhibit partial effects.

A typical vibration device utilizes the repulsive force of magnets. A magnet is disposed on a vibration plate at the midpoint of the height of the vibration device casing, and upper and lower magnets opposite to the magnet are disposed on the upper and lower surfaces of the casing, so that the magnetic field and wavelength generated by the vibration device affect human blood flow, which results in health improving effects.

The present invention is based on the shoe vibration device technology by the applicant and has been devised to apply it to mattresses or beds.

### DETAILED DESCRIPTION OF THE INVENTION

### Technical Problem

Therefore, it is an object of the present invention to provide a vibration device that is mounted inside a mattress or bed, has excellent vibration effects and can improve health and sleep through body vibration, and is durable and economical, and a mattress or bed mounted with this device.

### Technical Solution

In order to achieve the above object, an aspect of the present invention provides a vibration device mounted inside a mattress of a bed, wherein: the vibration device comprises a vibration element and a casing, and the vibration element comprises a first magnet mounted on a first frame, one end of which is fixed to the casing, the upper part of the casing is mounted with a second magnet opposite to the first magnet and exerting a repulsive force, and the lower part of the casing is mounted with a third magnet opposite to the first magnet and exerting a repulsive force.

The vibration element may comprise a fixing portion extending vertically with a predetermined width, having a slot formed therein and fixed to the casing, and the first frame comprises a first support frame extending long horizontally from the center of the first fixing portion toward the internal center of the casing.

A first branch branching upward and downward may extend from a position biased toward the fixing portion in the support frame, and a first magnet may be mounted at the end of the first branch.

A second branch branching upward and downward may extend from the tip end of the support frame or a position adjacent to the tip end, and a first magnet may be also mounted at the end of the second branch.

The first and second branches may be respectively formed with a symmetrical structure with respect to the first support frame, and may have a curved or linear shape.

Small recessed portion are formed on both sides of the first and second branch connection portions of each of the first and second support frames to form a neck portion with reduced width, and in each of the first and second branches, opposing both sides of the bent boundary portion are reduced to form a recess. A neck portion may be formed at the connection portion with the disk on which the first magnet is placed at the end of each branch, and neck portions may be formed on both sides of the portion where the fixing portion and the support frame are connected.

Another aspect of the present invention provides a vibration device mounted inside a mattress of a bed, wherein: the vibration device comprises a vibration element and a casing, and the vibration element comprises a first vibration element including a first magnet mounted on a first frame, one end of which is fixed to a side surface of the casing, and a second vibration element including a first magnet mounted on a second frame, one end of which is fixed to the opposite side surface of the casing; the first vibration element and the second vibration element are symmetrical with respect to the center of the casing and have the same weight, the upper part of the casing is mounted with a second magnet opposite to the first magnet and exerting a repulsive force, and the lower part of the casing is mounted with a third magnet opposite to the first magnet and exerting a repulsive force; the first vibration element and the second vibration element are spaced apart from each other with respect to the center of the casing, so that each of the first and second vibration elements has a cantilever-like vibration structure, vibrations of the casing are transmitted from both sides of the casing, generating vibrations uniformly to the casing as a whole.

The first vibration element may include a first fixing portion having one end fixed to the casing, and the vibration element may include a second fixing portion having one end fixed to the opposite side of the casing.

Yet another aspect of the present invention provides a vibration device comprising a vibration element and a casing, wherein: the vibration element comprises a first magnet mounted on a frame, one end of which is fixed to the casing, the upper part of the casing is mounted with a second magnet opposite to the first magnet and exerting a repulsive force, and the lower part of the casing is mounted with a third magnet opposite to the first magnet and exerting a repulsive force; the vibration element comprises a fixing portion extending vertically with a predetermined width, having a slot formed therein and fixed to the casing, and the frame comprises a support frame extending long horizontally from the fixing portion toward the center; a first branch branching upward and downward extends from a position biased toward the fixing portion in the support frame, and a first magnet is mounted at the end of the first branch; a second branch branching upward and downward extends from the tip end of the support frame or a position adjacent to the tip end, and a first magnet is also mounted at the end of the second branch; and the first and second branches are respectively formed with a symmetrical structure on the basis of the second support frame, and has a curved or linear shape.

Small recessed portions are formed on both sides of the first and second branch connection portions to form a neck portion with reduced width, and in the respective branches, opposing both sides of the bent boundary portion are reduced to form a recess. A neck portion may be formed at the connection portion with the disk on which the first magnet is placed at the end of each branch, and neck portions may be formed on both sides of the portion where the fixing portion and the support frame are connected.

A further aspect of the present invention provides a cushion comprising the above-mentioned vibration device.

A further aspect of the present invention provides a mat comprising the above-mentioned vibration device.

### Advantageous Effects

A bed comprising the vibration device of the present invention simultaneously applies magnetic force and vibrations that are beneficial to the human body, which helps improve blood flow and is excellent at relieving muscle pain. Furthermore, the bed absorbs impacts and vibrations from the human body to aid sleep and add health-improving functions, thereby significantly enhancing the marketability of beds, mattresses, and other bedding.

### BRIEF DESCRIPTION OF THE DRAWINGS

FIG. 1 is a diagram showing a vibration element of the present invention;
FIG. 2 is a diagram of a vibration device of the present invention comprising the vibration element of FIG. 1;
FIG. 3 is a diagram for explaining the vibration principle of the vibration device of the present invention;
FIG. 4 is a diagram showing a state in which the vibration device of the present invention is disposed on a bed; and
FIG. 5 is a diagram showing another embodiment of the vibration element of the present invention.

### DETAILED DESCRIPTION OF THE EMBODIMENTS

The present invention is susceptible to various modifications and alternative forms. Therefore, specific embodiments are illustrated in the drawings and specific details for carrying out the invention are described in detail. However, this is not intended to limit the present invention to the particular forms set forth, but on the contrary to cover all modifications, equivalents, and alternatives falling within the spirit and technical scope of the present invention.

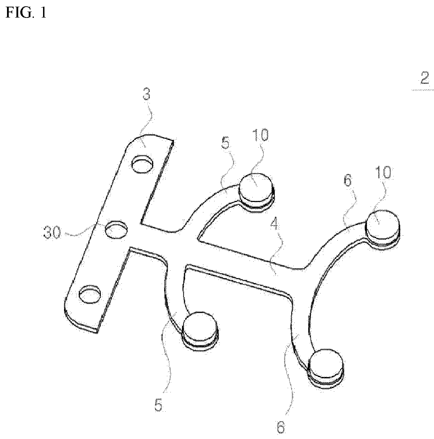

First, the structure of the vibration element 2 of the present invention will be described with reference to FIG. 1.

A narrow, long bar or band-shaped fixing portion 3 is formed on one side of the vibration element 2. The fixing portion 3 is preferably made with a length covering a significant portion of the side surface so that it can be firmly fixed to the side surface of the casing 100 described below. The fixing portion 3 is formed with a plurality of slots 30 for coupling to the casing described below.

A support frame 4 extending horizontally is formed with a length longer than the fixing portion 3 from the longitudinal center of the fixing portion 3. A first branch 5 extends from the support frame 4 at a position slightly biased toward the fixing portion 3. The first branch 5 is symmetrical with respect to the support frame 4. It has a shape similar to an arc, which is a portion of a circle, and has a curved bar or band structure that convexly extends toward the fixing portion 3. First magnets 10, which function as both a vibrator and a weight, are located at two points on the two ends of the first branch 5. Similarly, a second branch 6 extends from the end of the support frame 4. The second branch 6 has the same shape as the first branch 5. First magnets 10, which function as vibrators and weights, are located at two points on the ends of the second branch 5.

In the above description, the first branch 5 and the second branch 6 may be made in various shapes other than those shown. Furthermore, the two branches can be made in different shapes. The vibration element 2 is made integrally from a thin metal plate, excluding the magnetic portion to facilitate vibration. However, plastic materials with excellent elasticity can also be used. The magnet should be interpreted as a broad concept that includes not only pure magnets but also other structures for increasing weight or facilitating attachment.

The first magnets 10 are disposed in a total of four. The first magnets 10 are mounted on the upper surface of the branch tip end, but they can also be mounted on the lower surface, or can also be mounted on both upper and lower surfaces to increase repulsive force. Furthermore, if a highly magnetic material is used, they can be mounted in an embedding form in the end, or insert-molded integrally with the branch portion.

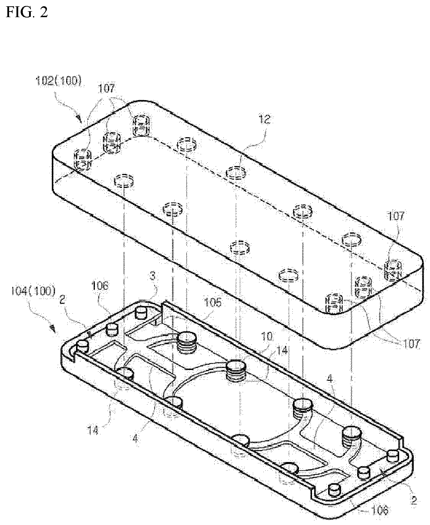

FIG. 2 is a diagram of a vibration device 1 of the present invention comprising the vibration element 2 of FIG. 1. The vibration element 2 is mounted in the casing 100. The casing 100 is made in the shape of a long box so that it can be easily mounted on a bed. The casing 100 consists of an upper casing 102 and a lower casing 104. The casing 100 is preferably made of a rigid plastic material to efficiently transmit vibrations.

Two vibration elements 2 are mounted on both sides of the casing 100. They are preferably suspended at mid-height from the casing 100 so as to maximize vibration effects. Therefore, in the present invention, the vibration element is suspended horizontally on the upper surface of the lower casing 104. The side surface edge 106 of the lower casing 104 has a thickness with width so as to couple with the fixing portion 3, and rods 106 protrude upward for insertion into the slots 30. The side surface of the upper casing 102 does not have a side surface with the same width as the side frame 106 of the lower casing 104, and instead is formed with a receiving protrusion 107, which has a receiving groove formed therein and faces each of the rods 106, into which the upper ends of the rods 106 are inserted.

The vibration element 2 is mounted in pairs on the left and right sides with the same structure and arrangement, and the first magnets 10 are formed in a total of eight. The first magnets 10 are arranged at approximately equal intervals while forming in pairs along the longitudinal direction of the casing 100. Further, all first magnets 10 are located biased above or below the casing 100. The structure in which each magnet are branched and biased from the central support frame 4 is advantageous for generating and transmitting vibration.

The present invention utilizes a repulsive force between magnets to ensure efficient vibration of the vibration element 2. For this purpose, a second magnet 12 is mounted in the upper casing 102 in a position opposite to the first magnet 10, and a third magnet 14 is mounted in the lower casing 104 in a position opposite to the first magnet 10. The magnetic pole of the exposed surface of the second magnet 12 facing downward is set to have the same polarity as the surface of the first magnet 10 opposite to the second magnet 12. Similarly, the magnetic pole of the exposed surface of the third magnet 14 facing upward is set to have the same polarity as the surface of the first magnet 10 opposite to the third magnet 12.

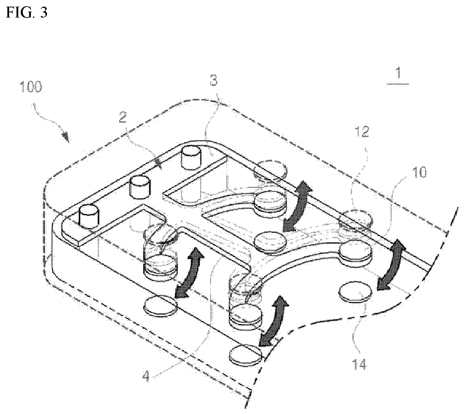

FIG. 3 is a diagram for explaining the vibration principle of the vibration device 1 of the present invention.

When sleeping in bed, twisting and turning or even slightly moving the body vibrates the vibration element 2 mounted inside the casing 100, causing the first magnet 10 to move upward or downward. When moving upward, the first magnet 10 is pushed downward by the second magnet 12, and moved further downward past the horizontal center line, it is then pushed by the third magnet 14 and moved upward. This up and down movement is repeated, causing fine, uniform vibrations. When the four first magnets 10 disposed on one vibration element 2 each vibrate and their vibration phases match, the waveforms overlap to cause constructive interference and amplify the waveform. Vibrations are transmitted to a whole casing 100 from the side surfaces via the support frame 4 and/or the fixing portion 3. In the present invention, two vibration elements 2 are disposed, so vibrations are transmitted effectively to the whole casing 100 from two vibration sources via the support frames 4 on both the left and right sides and the fixing portion 3.

One vibration element 2 disposed in the vibration device 1 is a single-sided fixed type in which the fixing portion 3 is a fixed shaft. Therefore, as the vibration element is closer to the center, the more eccentric acts, which is advantageous for generating vibration. When such vibration elements 2 are disposed to two, the two first magnets 10 located in the middle of the longitudinal direction as the whole vibration device 1 vibrate most strongly, and this vibration can be evenly transmitted through the two sides of the casing 100 via the support frame 4 and the fixed element 3. Furthermore, although it is very small, the repulsive force generated by the vibration of the first magnet 10 causes the second magnet 12 to vibrate slightly, thereby assisting the vibration.

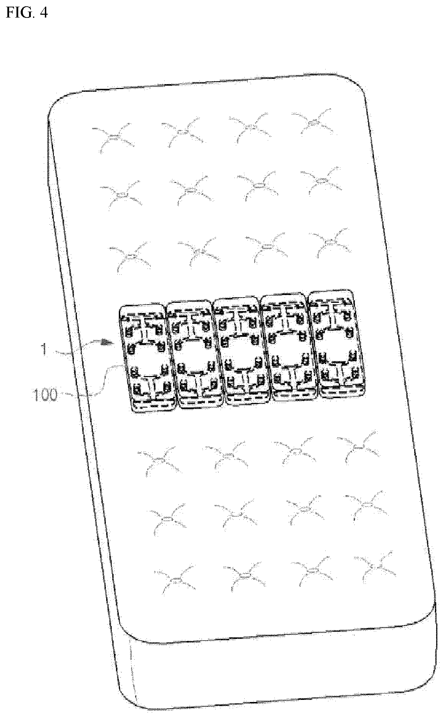

FIG. 4 is a diagram showing a state in which the vibration device 1 of the present invention is disposed on a bed. The vibration device 1 is dispose on an upper part of a bed mattress and can fully sense human presence and movement to generate vibrations. The vibration devices 1 are mounted in a plurality by matching rows and columns at the central area of the mattress, so that the long direction of the casing 100 is placed vertically. While the illustrated example shows one row and five columns, it goes without saying that vibration devices can be made in multiple rows and columns in accordance with the areas requiring health improvement.

The mounting area of the vibration device 1 can be concentrated on the entire mattress, as well as on the areas where the head or lumbar region is located to improve the health functions of specific parts of the body.

The vibration device 1 can be mounted in various ways, such as being embedded in a mattress or being built into a separate mat and then covering it over the mattress. Further, protruding magnetic material can be embedded adjacent to the vibration device 1 to enhance the health improving effects.

Those skilled in the art will understand that the vibration device 1 of the present invention can be applied to a variety of applications, including not only beds but also mattresses and cushions.

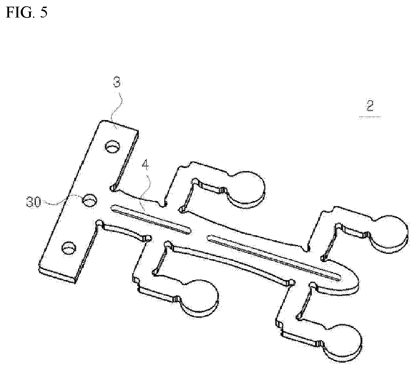

FIG. 5 is a diagram showing another embodiment of the vibration element 2 of the present invention.

The basic structure of the second embodiment is nearly identical to that of the first embodiment, but differs in that the length of the support frame 4 is slightly extended and a slot with a width equivalent to half the width is perforated along its entire length. This structure is advantageous for generating vibrations because it reduces the weight of the support frame 4.

Further, the first branch 5 and the second branch 6 are formed with a straight " 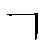 "-shaped bend or bending structure, rather than a curved one, and a first magnet 10 is mounted at each tip end. The second branch 6 is mounted biased adjacent to the end of the support frame 4 rather than at the end thereof.

One of the features of the second embodiment of the present invention lies in the formation of a concave neck or recess in the vibration element 2 to effectively generate vibration even with slight impacts or movements. Specifically, small recessed portions are formed on both sides of the connection portion between the support frame 4 and the first and second branches 5 and 6 to form a neck portion with reduced width, each branch is formed with a recess by narrowing the opposing both sides of the bent boundary portion, and a neck portion is formed at the connection point with the disk where the first magnet 10 is placed at the end of each branch. Furthermore, neck portions are formed on both sides of the initial portion where the fixing portion 3 and the support frame 4 are connected.

Such a structure reduces weight as a whole. Further, the neck portion or the recess portion is formed at the connection area between the components or at the area where direction changes, thereby acting like nodes, which helps to generate and transmit vibration more effectively.

The vibration element 2 of the present invention can appropriately select a variety of structures, in addition to those shown in the figures.

Although the present invention has been illustrated and described above with reference to preferred embodiments, it will be understood by those skilled in the art that the invention is not limited to the above embodiments, and various modifications and alterations can be made to the embodiments without departing from the spirit of the present invention. It is intended that the present invention encompass such modifications and alterations as fall within the scope of the appended claims.

## Claims

1. A vibration device mounted inside a mattress of a bed, wherein:
the vibration device comprises a vibration element and a casing, and the vibration device comprises a first vibration element disposed on one side of the casing and a second vibration element disposed on the other side opposite to the one side of the casing;
the first vibration element comprises a first magnet mounted on a first frame, one end of which is fixed to one side surface of the casing, the upper part of the casing is mounted with a second magnet opposite to the first magnet and exerting a repulsive force, and the lower part of the casing is mounted with a third magnet opposite to the first magnet and exerting a repulsive force;
the first vibration element comprises a first fixing portion extending vertically with a predetermined width, having a slot formed therein and fixed to one side surface of the casing, and the first frame comprises a first support frame extending long horizontally from the center of the first fixing portion toward the internal center of the casing;
a first branch branching upward and downward extends from a position biased toward the first fixing portion in the first support frame, and a first magnet is mounted at the end of the first branch;
a second branch branching upward and downward extends from the tip end of the first support frame or a position adjacent to the tip end, and a first magnet is also mounted at the end of the second branch;
the first and second branches are respectively formed with a symmetrical structure with respect to the first support frame, and has a curved or linear shape;
the first magnet disposed at the end of the first branch is located further inside than a branch point between the first support frame and the first branch, and the first magnet disposed at the end of the second branch is disposed further inside than a branch point between the first support frame and the second branch, so that the vibration of each first magnet is transmitted to the first fixing portion via the first branch and the first support frame, toward the outside of the casing based on the location of the first magnet;
the second vibration element comprises a first magnet mounted on a second frame, one end of which is fixed to the other side surface of the casing, the upper part of the casing is mounted with a second magnet opposite to the first magnet and exerting a repulsive force, and the lower part of the casing is mounted with a third magnet opposite to the first magnet and exerting a repulsive force;
the second vibration element comprises a second fixing portion extending vertically with a predetermined width, having a slot formed therein and fixed to the other side surface of the casing, and the second frame comprises a second support frame extending long horizontally from the center of the second fixing portion toward the center of the casing;
a first branch branching upward and downward extends from a position biased toward the second fixing portion in the second support frame, and a first magnet is mounted at the end of the first branch;
a second branch branching upward and downward extends from the tip end of the second support frame or a position adjacent to the tip end, and a first magnet is also mounted at the end of the second branch;
the first and second branches are respectively formed with a symmetrical structure on the basis of the second support frame, and has a curved or linear shape;
the first magnet disposed at the end of the second branch is located further inside than a branch point between the second support frame and the second branch, and the first magnet disposed at the end of the second branch is disposed further inside than a branch point between the second support frame and the second branch, so that the vibration of each first magnet is transmitted to the second fixing portion via the second branch and the second support frame, toward the outside of the casing based on the location of the first magnet; and
the first vibration element and the second vibration element are spaced apart in a symmetrical structure facing each other with respect to the internal center of the casing, so that the first and second fixing portions of one and the other sides of the casing of the vibration device serve as vibration sources, and vibration is transmitted to a whole casing.

2. The vibration device according to claim 1,
wherein small recessed portion are formed on both sides of the first and second branch connection portions of each of the first and second support frames to form a neck portion with reduced width, and in each of the first and second branches, opposite sides of the boundary portion are reduced to form a recess.

3. A cushion comprising the vibration device as set forth in claim 1.

4. A mat comprising the vibration device as set forth in claim 1.
